# EUROPÄISCHE PATENTANMELDUNG

(11) **EP 1 442 700 A1**
(43) Veröffentlichungstag der Anmeldung: **04.08.2004**
(21) Anmeldenummer: 03028209.9
(22) Anmeldetag: 09.12.2003
(51) Int. Cl.: A61B 5/022, A61B 5/0235

(54) **Blutdruckmessgerät**

(30) Priorität: 31.01.2003 DE 10303906
(71) Anmelder: ERKA. KALLMEYER Medizintechnik GmbH & Co. KG, 83646 Bad Tölz (DE)
(72) Erfinder: Götz, Peter, 83677 Greiling (DE); Ziel, Jörg, 83677 Greiling (DE); Wingender, Werner, 83677 Greiling (DE); von Benthen, Jochen, 83646 Bad Tölz (DE)
(74) Vertreter: Schwabe - Sandmair - Marx

(57) **Zusammenfassung**

Bei einem mechanischen Blutdruckmessgerät, das eine Messdose (1) mit einer Druckanzeige zur Messung eines Druckverlaufs und eine Bedieneinheit (4) mit einer Druckerzeugungsund einer Druckregulierungsvorrichtung (6, 7; 8) aufweist sind die Messdose (1) und die Bedieneinheit (4) lösbar miteinander verbunden. Vorzugsweise sind die Messdose (1) und die Bedieneinheit (4) in zwei zueinander um 180° verdrehten Stellungen miteinander verbindbar.

## Beschreibung

Die vorliegende Erfindung betrifft ein mechanisches Blutdruckmessgerät, insbesondere ein Gerät mit einer Bedieneinheit zur handbetriebenen Druckerzeugung und Druckregulierung.

Eine Art von mechanischen Blutdruckmessgeräten nach dem Stand der Technik umfassen eine Druckmanschette, die z. B. am Oberarm mittels eines Klettverschlusses befestigt wird, und ein Handgerät mit einer Bedieneinheit zur Druckerzeugung und Druckregulierung sowie mit einer Messdose, die ein Messwerk und eine Anzeige zur Angabe des Druckverlaufs aufweist. Zur Druckerzeugung in der Manschette ist diese über einen oder zwei Schläuche mit dem Handgerät verbunden und kann z. B. durch einen Pumpball an der Bedieneinheit durch den Anwender aufgepumpt werden. Gleichzeitig mit dem Druck der Manschette wird durch das Aufpumpen ein Druck in der Messdose erzeugt, der von dem Messwerk bestimmt und von der Druckanzeige angezeigt wird. Zur Druckregulierung weist das Handgerät z. B. ein Ventil an der Bedieneinheit auf, das beim Druckaufbau geschlossen ist und zur Messung des Blutdruckes geöffnet wird, so dass sich der Druck in der Manschette und in der Messdose langsam kontinuierlich verringert. Dabei ist eine sehr feine Einstellbarkeit des Ventils erforderlich, um eine exakte Druckregulierung vornehmen zu können, bei der ein Druck der Systole und der Diastole zu einem bestimmten Zeitpunkt deutlich erkannt werden können.

An der Bedieneinheit ist an einer Seite des Pumpballs ein festes Element wie etwa ein Löffel vorgesehen, der im Handballen liegt und gegen den der Pumpball mit den Fingern gedrückt werden kann. Dabei dient der Löffel bei der Druckerzeugung als stabilisierendes Element bei der Handhabung des Blutdruckmessgeräts. Auf einer dem Löffel gegenüberliegenden Seite, d.h. auf der anderen Seite des Druckballs, ist das Ventil vorgesehen, so dass dieses z. B. mit Zeigefinger und Daumen bedient werden kann, während der Pumpball mit dem Löffel weiterhin in dem Handballen gehalten wird. An einem vorderen Ende des Pumpballs ist die Messdose angeordnet von deren Anzeige eine Druckänderung abgelesen werden kann, während der Anwender den Pumpball in der Hand behält und das Ventil reguliert.

Ein solches Blutdruckmessgerät ist einhändig bedienbar. Um eine exakte und zuverlässige Blutdruckmessung zu gewährleisten ist es erforderlich, das Messwerk in der Messdose in regelmäßigen Abständen messtechnisch zu überprüfen. Hierfür wird das Blutdruckmessgerät in der Regel an den Hersteller oder einen von diesem autorisierten Reparateur geschickt, dort geprüft und kalibriert und wieder an den Anwender zurückgesandt.

Bei den beschriebenen herkömmlichen Blutdruckmessgeräten besteht der Nachteil, dass die Anordnung des Löffels, des Pumpballs, des Ventils und der Druckanzeige auf eine Bedienung mit der rechten Hand ausgelegt ist. Wird das Gerät von einem Linkshänder verwendet, der ebenfalls den Löffel im Handballen hält, den Pumpball mit den Fingern bedient und anschließend das Ventil mit Zeigefinger und Daumen reguliert, ist das Handgerät gegenüber einem rechtshändigen Gebrauch um 180° verdreht. Die Druckanzeige zeigt daher von dem Anwender weg und ist für diesen nicht ablesbar.

Es wurden daher Geräte entwickelt, bei welchen der Löffel an dem Druckball im Vergleich zu einer Anordnung für Rechtshänder auf einer gegenüberliegenden Seite angeordnet werden kann, so dass auch ein Linkshänder den Pumpball leicht gegen den Löffel drücken kann. Jedoch ist es dann kaum möglich, das Ventil mit derselben Hand zu bedienen, da dies auf der gleichen Seite wie der Löffel liegt und mit den Fingern nicht zugänglich ist. Ferner wurden Blutdruckmessgeräte entwickelt, die anstelle eines seitlich angeordneten Drehventils zur Druckregulierung einen Druckknopf aufweisen, der oben auf der dem Anwender zugewandten Seite des Handgerätes angeordnet ist, so dass dieser bei einem Wechsel von der rechten Hand zur linken Hand in gleicher Weise von oben bedient werden kann. Derartige Druckregulierungen sind für einen Arzt oder medizinischen Assistenten jedoch ungewohnt und weisen jedoch den Nachteil auf, dass eine Feineinstellung des Drucks nur schwer möglich ist.

Weiter ist es ein Nachteil bei den herkömmlichen Blutdruckmessgeräten, dass das gesamte Messgerät während der Durchführung der messtechnischen Kontrolle ausfällt. Für einen Anwender ist daher ein Zweitgerät erforderlich, um weiterhin eine regelmäßige Blutdruckkontrolle durchführen zu können.

Es ist eine Aufgabe der vorliegenden Erfindung, ein Blutdruckmessgerät zu schaffen, das leicht auf verschiedene Anwender und Anwendungssituationen einstellbar ist, eine flexible Handhabung ermöglicht, den Wartungsaufwand reduziert und Ausfallzeiten verringert oder sogar ausschließt.

Die Aufgabe wird durch ein Blutdruckmessgerät nach Anspruch 1 erfüllt. Vorteilhafte Ausgestaltungen eines erfindungsgemäßen Blutdruckmessgerätes gehen aus den Unteransprüchen hervor.

Demnach weist ein Blutdruckmessgerät eine Messdose mit einem Messwerk zur Messung eines Druckverlaufes und eine Druckanzeige z. B. in Form eines Skalenblattes, über das ein Zeiger gemäß dem anliegenden Druck dreht, auf. Weiter weist das Blutdruckmessgerät eine Bedieneinheit mit einer Druckerzeugungsvorrichtung, vorzugsweise einem latexfreien Pumpball, und einer Druckregulierungsvorrichtung auf, wie etwa ein Ablassventil, vorzugsweise aus Metall, z. B. aus verchromtem Messing. Durch das Ablassventil kann der Druckverlauf mittels eines Drehmechanismus fein eingestellt werden, so dass sogenannte Slip-Stick-Effekte vermieden werden können. Erfindungsgemäß sind die Messdose und die Bedieneinheit lösbar miteinander verbunden. D.h., in einem betriebsbereiten Zustand zur Messung des Blutdrucks sind die Messdose und die Bedieneinheit fest mit einander verbunden und bilden eine starre Einheit. Die Verbindung kann jedoch gelöst werden, so dass die Messdose und die Bedieneinheit gegeneinander beweglich sind. Dadurch können diese z. B. miteinander in Verbindung bleiben, aber gegeneinander verdreht oder verschoben werden.

Vorzugsweise sind die Messdose und die Bedieneinheit derart lösbar miteinander verbunden, dass sie vollständig voneinander getrennt, wieder zusammengesetzt und fest miteinander verbunden werden können.

Durch einen solchen erfindungsgemäßen Aufbau des Blutdruckmessgerätes wird es möglich, die Messdose von der Bedieneinheit abzunehmen und allein für Wartungsarbeiten, wie etwa für eine vorgeschriebene regelmäßige messtechnische Kontrolle, zur Verfügung zu stellen. Bei einem vollständigen Ausfall der Messdose z. B. durch irreparable Fehler beim Messwerk, ist es möglich, lediglich die Messdose durch eine neue funktionsfähige und geprüfte Messdose auszutauschen und die ursprüngliche Bedieneinheit weiter zu verwenden. Es ist auch denkbar eine Messdose derart kostengünstig herzustellen, dass sie als eine Art Einwegdose vorgesehen werden kann, die entsorgt wird und durch eine neue Messdose ersetzt wird sobald eine maßtechnische Kontrolle oder Reparaturen notwendig werden. In diesem Sinne bildet die Messdose eine Austauscheinheit, durch die eine kontinuierliche Einsatzfähigkeit des Blutdruckmessgerätes gewährleistet werden kann.

In einer bevorzugten Ausführungsform des erfindungsgemäßen Blutdruckmessgerätes sind die Messdose und die Bedieneinheit in wenigstens zwei verschiedenen vorbestimmten Stellungen zueinander miteinander verbindbar. Vorzugsweise ist bei einer Draufsicht auf die Anzeige der Messdose eine erste Stellung der Bedieneinheit zur Messdose für einen rechtshändigen Gebrauch und eine zweite Stellung der Bedieneinheit zur Messdose für einen linkshändigen Gebrauch geeignet. Bei einer Bedieneinheit, die z. B. eine kreuzförmige Halterung mit vier Anschlussenden umfasst, kann z. B. an einem Ende die Messdose angeordnet sein, so dass für einen Anwender die Anzeige ersichtlich ist, und an den gegenüberliegenden Enden kann der Pumpball angebracht werden. Durch diese derart in einer Linie angeordnete Messdose und den so angeordneten Pumpball wird eine Achse des Blutdruckmessgeräts definiert. An den quer zu dieser Achse verlaufenden Anschlussenden der Halterung kann an einem Ende, z. B. bei Draufsicht auf die Anzeige das rechte Ende, der Halterung ein Löffel und ein Anschluss für einen Manschettenschlauch angeordnet sein, wobei sich der Löffel auf dieser rechte Seite entlang dem Pumpball erstreckt. An dem gegenüberliegenden Anschlussende der Halterung, d. h. bei Draufsicht auf die Anzeige der Messdose an dem linken Ende, kann dem Löffel gegenliegend ein Ablassventil vorgesehen sein. In einer ersten Stellung ist demnach bei Draufsicht auf die Messdose der Pumpball gegenüberliegend der Messdose, das Ablassventil auf der linken Seite und der Löffel, bzw. der Manschettenschlauch auf der rechten Seite angeordnet. In einer bevorzugten Ausführungsform des Blutdruckmessgeräts ist in einer zweiten Stellung die Bedieneinheit gegenüber der Messdose um 180° gegenüber der oben beschriebenen ersten Stellung entlang der Achse verdreht, welche durch die lineare Anordnung der Messdose und des Pumpballs gebildet wird. Demnach verbleiben die Messdose und der Pumpball in einer solchen zweiten Stellung in ihrer Position. Die Positionen des Löffels und des Ablassventils sind jedoch miteinander vertauscht, so dass bei Draufsicht auf die Anzeige der Löffel auf einer linken Seite des Pumpballs und das Ventil auf einer rechten Seite angeordnet sind. Dabei ist es vorteilhaft, wenn der Pumpball rotationssymmetrisch zur oben beschriebenen Achse ausgebildet ist, so dass er bei einer Drehung seine Anordnungsform nicht ändert. Natürlich ist es auch denkbar andere vorbestimmte Stellungen der Messdose und der Bedieneinheit zueinander vorzusehen, wie etwa um 90° um oben beschriebene Achse verdrehte Stellungen. Es können auch mehr als zwei verschiedene vorbestimmte Stellungen, wie etwa 4 oder 8 Stellungen, vorgesehen werden.

Das erfindungsgemäße Blutdruckmessgerät ist durch die Möglichkeit, die Messdose und die Bedieneinheit in unterschiedlichen Stellungen zueinander fest miteinander zu verbinden, bzw. eine feste Verbindung einer bestimmten Stellung zu lösen und die Messdose und die Bedieneinheit in einer anderen Stellung fest miteinander zu verbinden, auf einfache Weise an unterschiedliche Bedienanforderungen anpassbar. Insbesondere ist es möglich, das Blutdruckmessgerät problemlos auf die besonderen Anforderungen von Rechts- oder Linkshändern anzupassen, ohne auf eine Bedienfreundlichkeit des Geräts verzichten zu müssen.

Es kann eine Führungseinrichtung zur Führung der Messdose oder der Bedieneinheit in die wenigstens zwei vorbestimmten Stellungen vorgesehen sein. Hierfür kann z. B. an der Messdose eine Führungsrinne oder Führungsfläche angeordnet sein, auf welche die Bedieneinheit z.B. mit einem Anschlussende aufgelegt wird und entlang dieser Führungseinrichtung in die vorbestimmte Stellung gebracht wird. Es ist auch denkbar, als Führungseinrichtung ein erstes Führungsmittel an der Messdose und ein zweites Führungsmittel an der Bedieneinheit vorzusehen, wobei das erste und zweite Führungsmittel beim Zusammensetzen der Messdose und der Bedieneinheit zusammenwirken. Bei einer Ausführungsform der Erfindung weist die Messdose ein flaches zylinderförmiges Gehäuse auf, das auf seiner seitlichen Umfangsfläche mit einer Einlassöffnung zum Anschluss an das Drucksystem bzw. an die Bedieneinheit versehen ist. Als Führungseinrichtung ist um die Einlassöffnung eine in Umfangsrichtung zur Öffnung leicht gewölbte Auflagefläche vorgesehen. Zur Verbindung der Messdose mit der Bedieneinheit kann ein Anschlussende in die Einlassöffnung der Messdose eingesetzt werden. Hierfür kann das Anschlussende auf der Auflagefläche der Messdose aufgelegt werden und solange in Richtung der Öffnung verschoben werden bis ein Anschlag erfolgt. Grundsätzlich sind auch andere Führungseinrichtungen denkbar, wie etwa ein Stift an der Bedieneinheit der in eine Führungsrille an der Messdose eingreift oder ein Drehmechanismus mit verschiedenen Anschlag- oder Rastpositionen, so dass die Messdose und die Bedieneinheit bei einem Anschlag oder Einrasten in einer vorbestimmten Stellung zueinander angeordnet sind.

In einer Ausführungsform der Erfindung sind die Messdose und die Bedieneinheit durch eine Schnapp-, Rast- oder Steckverbindung miteinander verbindbar, die erfindungsgemäß lösbar ausgebildet sind. Hierfür kann z. B. eine Schnappeinrichtung an der Messdose und eine Schnappgegeneinrichtung an der Bedieneinheit vorgesehen sein. Beim Zusammenführen der Messdose und der Bedieneinheit mittels der Führungseinrichtung können die Schnapp- und die Schnappgegeneinrichtung aufeinander zugeführt werden bis sie in einer Endstellung, d.h. in einer vorbestimmten Stellung zueinander, ineinander schnappen.

Für eine einfache Handhabung ist bei einer bevorzugten Ausführungsform des erfindungsgemäßen Blutdruckmessgerätes für die Verbindung der Messdose und der Bedieneinheit ein abnehmbares Verriegelungselement vorgesehen. Die Messdose und die Bedieneinheit werden dabei in einer vorbestimmten Stellung zueinander aneinandergefügt und mit Hilfe des Verriegelungselements miteinander lösbar verbunden. Hierfür sind sowohl an der Messdose als auch der Bedieneinheit z. B. Schnapp-, Rast- oder Steckeinrichtungen vorgesehen, in die Schnapp-, Rast- oder Steckgegeneinrichtungen eingreifen, die an dem abnehmbaren Verriegelungselement angeordnet sind. Dabei ist es vorteilhaft, z.B. an der Bedieneinheit mehrere dieser Einrichtungen an verschiedenen Stellen an der Bedieneinheit vorzusehen, die zu verschiedenen vorbestimmten Stellungen zwischen Messdose und Bedieneinheit gehören. Beispielsweise kann an einem Anschlussende der Bedieneinheit eine erste Schnapp- oder Rasteinrichtung auf einer Oberseite und eine zweite Schnapp- oder Rasteinrichtung auf einer Unterseite angeordnet sein, die der Oberseite gegenüberliegt. So kann die Bedieneinheit sowohl in einer ersten Stellung, als auch in einer hierzu um 180° gedrehten zweiten Stellung durch das Verriegelungselement an der Messdose angebracht werden. Durch das Verriegelungselement werden daher die Messdose und die Bedieneinheit in einer ausgewählten Stellung fest miteinander in einer ausgewählten Stellung verbunden. Wird das Verriegelungselement abgenommen, können die Messdose und die Bedieneinheit gegeneinander bewegt werden, und z. B. in eine andere vorbestimmte Stellung zueinander gebracht oder vollständig voneinander getrennt werden. Die Einstellung der Messdose gegenüber der Bedieneinheit oder das Austauschen der Messdose kann dadurch auf rasche Weise erfolgen, ohne dass hierfür ein weiteres Werkzeug erforderlich wird.

Zwischen der Messdose und der Bedieneinheit kann in dem Verbindungsbereich ein elastisches Element angeordnet werden, an dem sowohl die Messdose als auch die Bedieneinheit in der lösbar festen Stellung anliegen. Dadurch kann z.B. eine spielfreie Verbindung zwischen der Messdose und der Bedieneinheit ermöglicht werden, indem die Messdose und die Bedieneinheit gegen das elastische Element gepresst werden, wobei zwischen der Messdose und der Bedieneinheit eine axiale Vorspannung entstehen kann. Hierfür kann z.B. in der Einlassöffnung der Messdose ein elastischer Ring, etwa aus Gummi oder Kunststoff, eingesetzt werden, an dem das Anschlussende der Bedieneinheit nach dem Einführen in die Öffnung anliegt. Bei der Verbindung der Messdose mit der Bedieneinheit, z.B. durch das abnehmbare Verriegelungselement, kann die Bedieneinheit gegen den Ring gepresst werden, so dass eine feste vorgespannte Verbindung zwischen der Messdose und der Bedieneinheit entsteht.

In einer bevorzugten Ausführungsform ist an dem Blutdruckmessgerät eine Kennzeichnungseinrichtung zur Individualisierung des Geräts vorgesehen. Die Zugehörigkeit des Geräts zu einem Anwender ist dadurch leicht und sofort erkennbar. Für einen Linkshänder ist es z.B. möglich, ein an seine Anforderungen angepasstes Blutdruckmessgerät aus mehreren Geräten sofort herauszufinden. Bevorzugt ist die Kennzeichnungseinrichtung mit dem Verriegelungselement anbringbar. Dadurch kann bei der Anpassung des Geräts an einen Anwender gleichzeitig die Kennzeichnungseinrichtung angebracht werden. Hierfür weist das Verriegelungselement vorzugsweise einen plattenförmigen Bereich auf und ist zumindest teilweise transparent ausgebildet, so dass zwischen dem Verriegelungselement und der Bedieneinheit, bzw. der Messdose, eine Kennzeichnungseinrichtung, z. B. in Form einer beschrifteten oder markierten Einlage angeordnet werden kann. Dabei wird vorteilhafterweise das abnehmbare Verriegelungselement auf der oberen Seite, d. h. der Seite mit der Druckanzeige des Messgeräts angeordnet, so dass die Kennzeichnung bei einer beliebigen Stellung der Bedieneinheit gegenüber der Messdose immer von oben ablesbar ist. Es ist natürlich auch möglich, die Kennzeichnung unabhängig von der Verbindung der Messdose und der Bedieneinheit an einer gut sichtbaren Stelle an dem Blutdruckmessgerät anzubringen.

Bei dem Blutdruckmessgerät weist das Ablassventil zur Regulierung des Drucks in der Messdose eine Ablassschraube auf, die vorteilhaft durch eine Rastverbindung am Ventil gesichert ist, die eine mit Kraft überdrehbare Rastung bildet. Durch die Rastverbindung kann die Ablassschraube abgenommen und ersetzt werden, ohne dass ein weiteres Werkzeug erforderlich ist. Als Rastelement kann z. B. ein Splint in Form eines Federbogens verwendet werden, der um ein Zylinderelement des Ventils gespannt ist, auf das die Ablassschraube aufgeschoben wird. Der Splint greift in eine Nut auf dem Umfang des Zylinderelement ein und weist eine radial nach innen ausgebildete Nase auf, die durch ein Loch in der Nut des Zylinderelements in ein Loch eines Regulierelements eingreift, so dass diese verdrehsicher miteinander verbunden sind. Die Ablassschraube wird über den Federbogen geschoben, so dass zwischen dem Zylinderelement und der Ablassschraube eine Rastverbindung gebildet wird.

Der Pumpball, der die Druckerzeugungsvorrichtung an der Bedieneinheit bildet, weist an einem Ende eine Öffnung mit einem nach innen gestülpten Bereich auf, mit dem er auf einem hülsenförmigen Anschlussende der Halterung der Bedieneinheit angeordnet ist. Dabei umschließt der nach innen gestülpte Bereich das Anschlussende luftdicht. Hierfür kann der Pumpball z. B. aus Kunststoffmaterial gefertigt sein, das im Schleuderverfahren verarbeitet werden kann. Im Gegensatz zu einem Tauchverfahren, bei dem ein nach außen gestülpter Verbindungsbereich an dem Pumpball entsteht, ist bei dem Schleuderverfahren eine Formgebung des Pumpballs mit einem nach innen gestülpten Verbindungsbereich möglich. Mit einem innen liegenden Verbindungsbereich ist ein stabile Verbindung zu der Halterung der Bedieneinheit möglich und die Bedieneinheit kann insgesamt kürzer gebaut werden.

Die Sicherung der Ablassschraube durch ein Klemmelement an dem Ventil und ein Pumpball mit nach innen gestülpten Verbindungsbereich bilden vorteilhafte Ausgestaltungen eines Blutdruckmessgerätes, die auch unabhängig von den übrigen Merkmalen der Erfindung einsetzbar sind.

Ein erfindungsgemäßes Blutdruckmessgerät wird anhand eines Ausführungsbeispiels mit Hilfe der Zeichnung näher erläutert. In dieser stellen dar:
- Fig. 1: eine perspektivische Explosionszeichnung einer bevorzugten Ausführungsform eines erfindungsgemäßen Blutdruckmessgeräts,
- Fig. 2: Schnittansicht durch eine Halterung einer Bedieneinheit nach einer bevorzugten Ausführungsform der Erfindung,
- Fig. 3: Aufsicht auf ein Gehäuseteil einer Messdose gemäß einer bevorzugten Ausführungsform der Erfindung,
- Fig. 4: perspektivische Ansicht eines Löffels der Bedieneinheit in einer bevorzugten Ausführungsform,
- Fig. 5: seitliche Ansicht eines abnehmbaren Verriegelungselements nach einer bevorzugten Ausführungsform der Erfindung und
- Fig. 6: Schnittdarstellung durch einen Pumpball einer Bedieneinheit nach einer bevorzugten Ausführungsform eines Blutdruckmessgeräts.

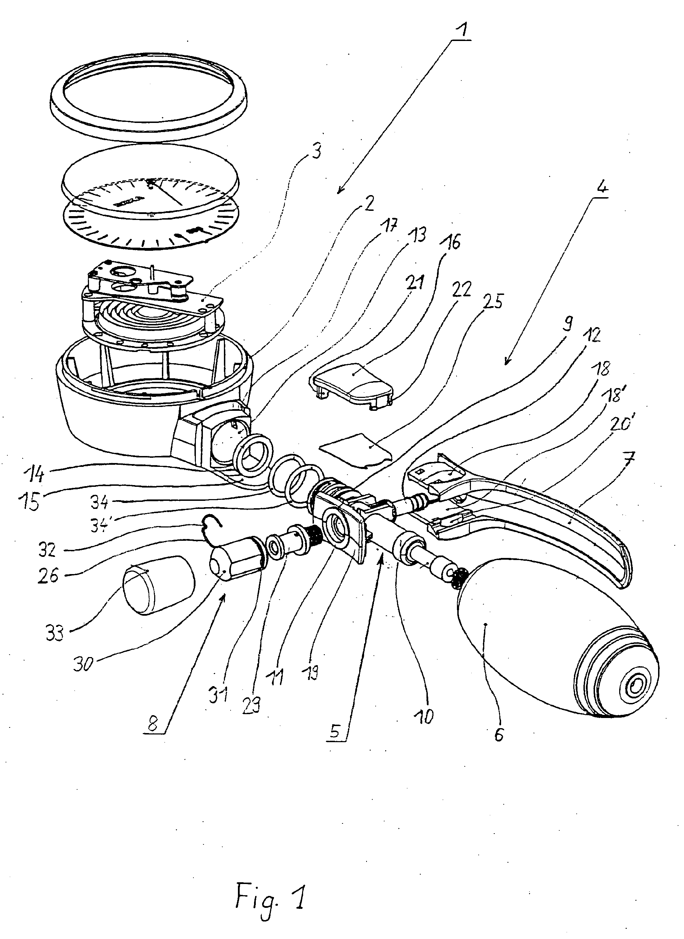

In Fig. 1 ist eine Explosionszeichnung mit den wesentlichen Einzelteilen eines Blutdruckmessgerätes nach einer bevorzugten Ausführungsform der Erfindung gezeigt. Das Blutdruckmessgerät weist eine Messdose 1 mit einem Gehäuseteil 2, einem Messwerk 3 sowie mit einer Skala, einer transparenten Abdeckung und einem Verschlussring auf. Das Gehäuseteil ist als zylindrisches Element mit einer im Vergleich zum Durchmesser geringen Höhe ausgebildet, so dass sich eine flache Dose ergibt. In der Umfangsfläche weist das Gehäuseteil 1 eine Anschlussöffnung 13 auf, mit der die Messdose 1 an eine Druckerzeugung des Blutdruckmessgeräts angeschlossen werden kann. Eine Bedieneinheit 4 des Blutdruckmessgerätes weist eine Halterung 5 auf, an der eine Druckerzeugungsvorrichtung, die einen Pumpball 6 und einen Löffel 7 umfasst, und eine Druckregulierungsvorrichtung in Form eines Ablassventils 8 angebracht sind.

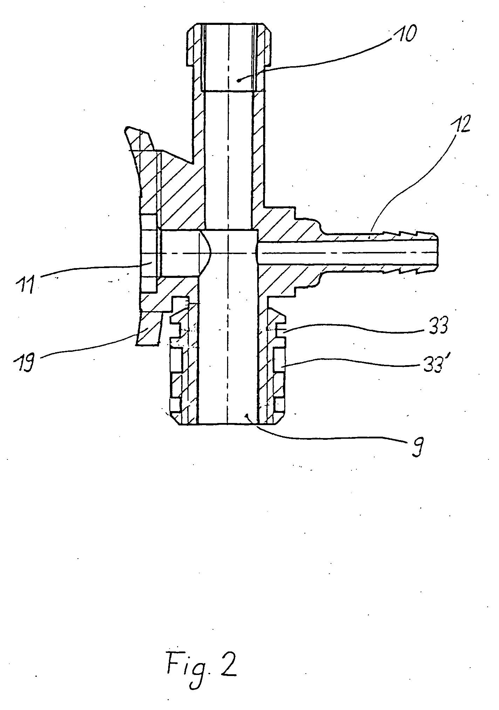

Die Halterung 5 ist, wie in Fig. 2 dargestellt, als kreuzförmiges Trägerelement mit vier Anschlussenden 9, 10, 11 und 12 ausgebildet, die im Inneren der Halterung 5 durch einen Hohlraum miteinander verbunden sind. Die Anschlussenden 9, 10, 11 und 12 weisen eine Art Röhrenform auf und sind nach außen offen. An dem Ende 9 der Halterung wird die Messdose 1 mittels der Anschlussöffnung 13 angesetzt und an dem Ende 10, das dem Ende 9 gegenüberliegt, wird der Pumpball 6 befestigt. Die Halterung 5 bildet entlang den Enden 9 und 10 eine Rotationsachse des Blutdruckmessgeräts, die im wesentlichen senkrecht auf die Umfangsfläche des Gehäuseteils 2 trifft. Quer zu den Enden 9 und 10 verlaufen die sich gegenüberliegende Enden 11 und 12 der Halterung 5. An dem Ende 11 wird das Ablassventil 8 und an dem Ende 12 wird der Löffel 7 angesetzt. Der Löffel 7 verläuft annähernd senkrecht zum Ende 12 im Wesentlichen parallel zur Rotationsachse des Blutdruckmessgerätes. Dabei ist der Löffel 7 derart gebogen, dass er seitlich an einer Oberfläche des Pumpballs 6 anliegt. An dem Ende 12 der Halterung 5 kann auch ein Schlauch (nicht gezeigt) angesetzt werden, der zu einer Manschette verläuft, die an einem Oberarm zur Blutdruckmessung angebracht wird. Der Hohlraum im Inneren der Halterung 5 stellt eine Verbindung zwischen dem Pumpball 6, der Messdose 1 und der Manschette her, so dass diese Teile drucktechnisch miteinander verbunden sind. Durch den Pumpball 6 kann ein Druck in der Manschette und in der Messdose 1 aufgebaut werden. Durch das Ablassventil 8, das in das Ende 11 der Halterung 5 eingesetzt ist und diese verschließt, kann der Druck reguliert werden.

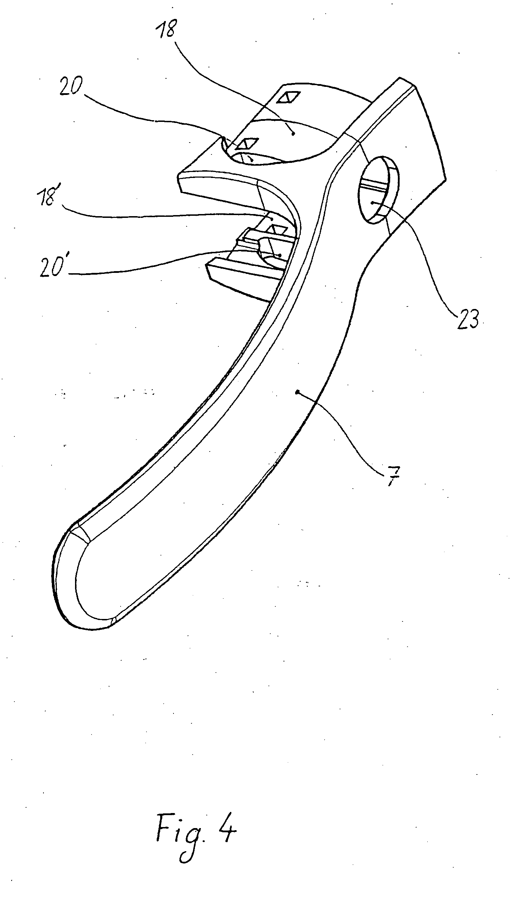

Der Löffel 7 der Bedieneinheit 4 ist mit zwei parallelen sich gegenüberliegenden Flächen 18 und 18' ausgebildet, wie Fig. 4 zeigt, zwischen welchen die Halterung 5 zu liegen kommt, wobei in einem die Flächen 18 und 18' verbindenden Löffelbereich eine Öffnung 23 vorgesehen ist, durch die das Ende 9 der Halterung 5 geführt wird. Das Ende 11 der Halterung 5 ist von einer senkrecht zum Ende 11 verlaufenden Platte 19 umgeben. Die Flächen 18 des Löffels 7 umfassen die Halterung 5 derart, dass sie mit der Platte 19 abschließen. Der Löffel 7, die Flächen 18 und die Platte 19 bilden daher eine Art Gehäusebereich für die Halterung 5.

Nach einer bevorzugten Ausführungsform der Erfindung ist es möglich, die Bedieneinheit 4 in verschiedenen vorbestimmten Stellungen an der Messdose 1 anzubringen. In einer ersten Stellung, wie in Fig. 1 dargestellt, liegt der Löffel 7 der Bedieneinheit 4 auf der rechten Seite des Pumpballs 6 und das Ablassventil 8 auf der linken Seite. In einer zweiten Stellung, ist die Bedieneinheit 4 z.B. um ein 180° um die von den Enden 9 und 10 der Halterung 5 gebildete Rotationsachse gegenüber der Messdose 1 gedreht, so dass der Löffel 7 auf der linken Seite und das Ablassventil 8 auf der rechten Seite des Pumpballs 6 angeordnet sind.

Das Gehäuseteil 2 der Messdose 1 wird zur Verbindung der Messdose 1 mit der Bedieneinheit 4 mit seiner Anschlussöffnung 13 auf das Ende 9 der Halterung 5 aufgesetzt. Hierfür sind auf dem Ende 9 Nuten 33 und 33' vorgesehen (Fig. 2), auf die O-Ringe 34 und 34' zur Herstellung einer Verbindung zwischen Messdose 1 und Bedieneinheit 4 aufgesetzt werden können. Beim Einführen des Endes 9 in die Anschlussöffnung 13 kommen die O-Ringe 34 und 34' derart in der Anschlussöffnung 13 zu liegen, dass die Messdose 1 und die Bedieneinheit 4 formschlüssig miteinander verbunden sind. Dabei dichten die O-Ringe 34 und 34' die Verbindung druckdicht ab. Beim Aufbringen einer Zugkraft wird der Formschluss gelöst und die Messdose 1 kann von der Bedieneinheit 4 abgenommen werden. Die Ringe 34 und 34' erzeugen daher eine Art lösbarer Reibverbindung zwischen Messdose 1 und Bedieneinheit 4. Zwischen dem Gehäuseteil 2 und dem Ende 9 der Halterung 5 kann vorzugsweise ein elastisches Element z. B. in Form eines Gummirings 14 eingesetzt werden. Der Gummiring 14 dient zur Herstellung einer spielfreien Verbindung zwischen Messdose 1 und Bedieneinheit 4.

Im Bereich des Bodens des Gehäuseteils 2 ist an der Anschlussöffnung 13 eine Führungseinrichtung in Form einer senkrecht von der Öffnung hervorstehenden Zunge 15 ausbildet. Die Zunge 15 ist in Annäherung an die kreisrunde Anschlussöffnung 13 gewölbt um diese Öffnung ausgebildet. Das Ende 9 der Halterung 5 kann entlang der Zunge 15 in die Anschlussöffnung 13 geführt werden.

Um die Messdose 1 mit der Bedieneinheit 4 in einer ausgewählten Stellung zueinander zu verriegeln ist ein abnehmbares Verriegelungselement 16 vorgesehen, das eine Verbindungseinrichtung 21, 22 sowohl für die Messdose 1 als auch für die Bedieneinheit 4 aufweist. Natürlich ist es auch möglich die Messdose 1 und die Bedieneinheit 4 durch eine geeignete Verbindung direkt, ohne weitere Elemente, miteinander zu verbinden. Zur Verbindung mit dem Verriegelungselement 16 weist das Gehäuseteil 2 der Messdose 1 auf einer der Zunge 15 gegenüberliegenden Seite der Anschlussöffnung 13, d. h. auf der Seite der Anzeige, eine Rasteinrichtung 17 auf.

In den Flächen 18 und 18' der Halterung 5, sind Rasteinrichtungen 20 und 20' vorgesehen. Das Verriegelungselement 16 ist teilweise plattenförmig ausgebildet und weist an sich gegenüberliegenden Seiten senkrecht abragende Rastgegeneinrichtungen 21 und 22 auf. Die Rastgegeneinrichtung 21 des Verriegelungselements 16 wirkt mit der Rasteinrichtung 17 an dem Gehäuseteil 2 der Messdose 1 und die Rastgegeneinrichtung 22 wirkt mit der Rasteinrichtung 20, bzw. 20', an einer Fläche 18, bzw. 18', zusammen, wenn die Messdose 1 und die Bedieneinheit 4 aneinander gesetzt sind. Dabei entspricht eine Rastverbindung mit der Fläche 18 einer ersten vorbestimmten Stellung zwischen Messdose 1 und Bedieneinheit 4 für einen rechtshändigen Gebrauch des Messgeräts und eine Rastverbindung mit der Fläche 18' einer zweiten vorbestimmten Stellung für einen linkshändigen Gebrauch, die gegenüber der ersten Stellung um 180° um die von den Enden 9 und 10 gebildete Achse der Halterung 5 gedreht ist. Das Verriegelungselement 16 greift sowohl in die Rasteinrichtung 17 als auch in die Rasteinrichtung 20, bzw. 20', ein und bildet eine feste Verbindung zwischen der Messdose 1 und der Bedieneinheit 4, wobei es diese in der vorbestimmten Stellung verriegelt. Dabei wird die Bedieneinheit 4 gegen den Gummiring 14 gepresst, so dass eine spielfreie Verbindung entsteht. Bei einer derartigen Anordnung kommt die Zunge 15 auf der gegenüberliegenden Seite des Verriegelungselements 16 auf der Fläche 18 oder 18' zu liegen, die nicht zur Herstellung der Verbindung verwendet wird. Insgesamt wird ein Gehäuse des Blutdruckmessgerätes daher gebildet aus dem Gehäuseteil 2 mit der Zunge 15, dem Verriegelungselement 16, dem Löffel 7 und der Platte 19. Das Verriegelungselement 16 ist aus der Rastverbindung lösbar und kann von dem Blutdruckmessgerät abgenommen werden. Dadurch kann die Bedieneinheit 4 von dem Gehäuseteil 2 der Messdose 1 gelöst werden und z. B. in einer um 180° gedrehten Stellung an dem Gehäuseteil 2 angebracht werden.

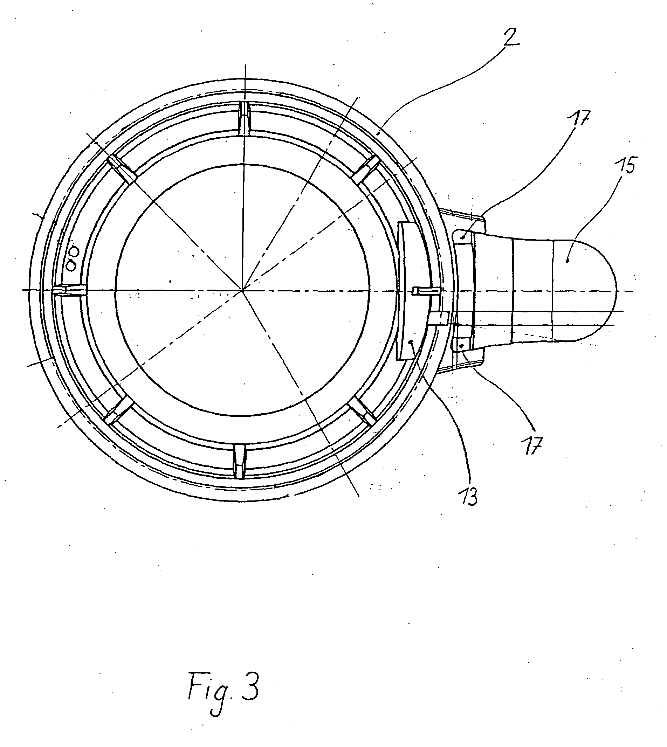

Die Rastverbindung der bevorzugten Ausführungsform der Erfindung ist in den Figuren 3, 4 und 5 an den Einzelelementen gezeigt. In Fig. 3 ist das Gehäuseteil 2 mit der Rasteinrichtung 17 in Form von Vertiefungen gezeigt, die zu beiden Seiten der Anschlussöffnung 13 senkrecht zur Fläche der Anzeige vorgesehen sind. In Fig. 4 ist der Löffel 7 mit den Flächen 18 und 18' gezeigt, in welchen jeweils eine Rasteinrichtung 20 in Form einer länglichen Aussparung vorgesehen ist.

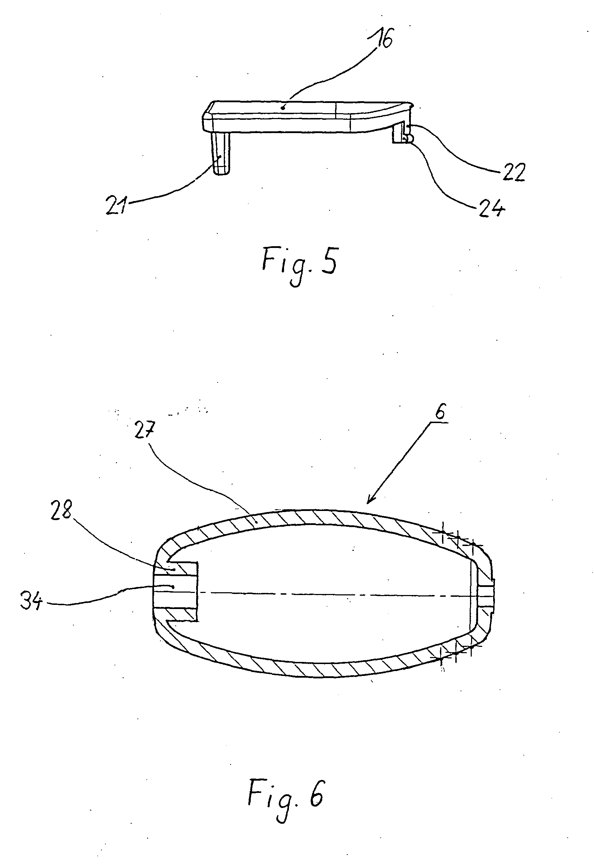

In Fig. 5 ist das teilweise plattenförmige Verriegelungselement 16 gezeigt. An einem Ende des Verriegelungselements 16 ist zu beiden Seiten senkrecht von der plattenförmigen Fläche abragend eine Rastgegeneinrichtung 21 in Form von zwei Fortsätzen angeordnet. Auf der gegenüberliegenden Seite des Verriegelungselements 16 ist eine Rastgegeneinrichtung 22 angeordnet, die aus zwei Nasen besteht, welche in die gleiche Richtung wie die Fortsätze 21 abragen. Zur Herstellung der Verbindung zwischen der Messdose 1 und der Bedieneinheit 4 wird das Verriegelungselement 16 mit den Fortsätzen 21 in die Vertiefungen 17 des Gehäuseteils 2 eingesetzt. Dabei greift eine Querverbindung zwischen den Fortsätzen 21 in eine Nut 35 am Ende 9 der Halterung 5 ein (siehe Figur 2). Mit den Fortsätzen 22 wird das Verriegelungselement 16 in die Aussparung 20 oder 20' an einer Fläche 18 oder 18' des Löffels 7 eingedrückt. Dabei geraten die Nasen 22 unter Vorspannung, so dass sie mit einem Wulst 24 am abstehenden Ende der Nasen 22 in die Aussparung 20 einrasten. Das Blutdruckmessgerät ist dann betriebsbereit. Soll die Messdose 1 ausgetauscht werden, kann das Verriegelungselement 16 abgenommen werden, so dass die Verbindung zwischen Messdose 1 und Bedieneinheit 4 gelöst werden kann.

An dem Blutdruckmessgerät kann eine Kennzeichnungseinrichtung 25 zur Individualisierung des Geräts für einen bestimmten Anwender angebracht werden, indem zwischen der Fläche 18, bzw. 18', und dem Verriegelungselement 16 z. B. ein Papierplättchen 25 eingelegt wird. Auf dem Papierplättchen kann z. B. der Name eines Anwenders oder eine farbige Markierung für eine Zugehörigkeit zu einer bestimmte Abteilung aufgedruckt werden. Das Verriegelungselement 16 ist bevorzugt transparent ausgebildet, so dass die Kennzeichnung durch das Verriegelungselement 16 abgelesen werden kann. Es ist auch möglich, nicht das gesamte Verriegelungselement transparent auszubilden, sondern nur ein Fenster über der Fläche der Kennzeichnungseinrichtung 25 vorzusehen. Das Verriegelungselement 16 erfüllt somit die Funktion der Kennzeichnung des Blutdruckmessgerätes, der Verbindung der Messdose 1 mit der Bedieneinheit 4 und bildet einen Gehäuseteil des Blutdruckmessgeräts.

Das Ablassventil 8 der Bedieneinheit 4 wird mittels eines zylindrischen Ventilhalses 29, auf den eine Kordelschraube 30 aufgesetzt ist, in das Ende 11 der Halterung 5 eingedrückt, wodurch eine druckdichte Verbindung entsteht. Dabei ist vorteilhafterweise an einem dem Ende 11 nahegelegenen Umfangsbereich der Kordelschraube 30 eine Nut 31 mit einem Loch durch die Wand der Kordelschraube vorzusehen. In dieser Nut 31 wird eine Art Federbogen 26 eingesetzt, der durch seine Vorspannung die Kordelschraube 30 umgreift. Der Federbogen weist eine radial nach innen ausgebildete Nase 32 auf, die durch das Loch in der Nut 31 der Kordelschraube 30 hindurch in eine Vertiefung in dem Ventilhals 29 eingreift und dadurch die Kordelschraube 30 gegenüber dem Ventilhals 29 verdrehsicher anbringt. Eine hülsenartige Ablassschraube 36 kann an ihrem Innenumfang eine Nut aufweisen, die beim Aufsetzen der Ablassschraube 36 auf die Kordelschraube 30 über dem Federbogen 26 zu liegen kommt, so dass die Ablassschraube 36 durch eine Rastverbindung mit der Kordelschraube 30 verbunden wird. Dadurch ist die Ablassschraube 36 zur Regulierung des Drucks über das Ablassventil 8 fest verbunden, kann jedoch von der Kordelschraube 30 abgezogen werden, so dass der Federbogen 26 gelöst werden kann und das Ablassventil 8 insgesamt ohne zusätzliches Werkzeug für Wartungsarbeiten demontiert werden kann.

In Fig. 6 ist ein ovaler Pumpball 6 mit einer elastischen Wand 27 gezeigt, der an einem Ende eine Öffnung 34 aufweist, in die das Ende 10 der Halterung 5 eingesetzt werden kann. Für eine erleichterte Montage und eine kürzere Bauweise des Bedienelements ist ein Wandbereich 28, der die Öffnung 34 umgibt, nach innen in den Pumpball hinein gestülpt ausgebildet. Neben einer erleichterten Montage ist es bei einem nach innen gestülpten Wandbereich 28 auch vorteilhaft, das zwischen den Flächen 18 und 18' des Löffels 7 keine Bereiche des Pumpballs 6 zu liegen kommen, so dass dieser von der Halterung 5 abgenommen werden kann ohne, dass weitere Elemente der Bedieneinheit 4 von der Halterung 5 abgenommen werden müssen.

Die Erfindung wurde anhand eines Ausführungsbeispiels erläutert. Es ist jedoch klar, dass die wesentlichen Merkmale der Erfindung auch durch eine andere Ausgestaltung einzelner Elemente des Blutdruckmessgerätes erfüllt werden können. Insbesondere kann anstatt einer Rastverbindung zwischen der Messdose 1 und der Bedieneinheit 4 auch eine Schraubverbindung verwendet werden. Ferner ist es natürlich möglich mehr als zwei vorbestimmte Stellungen der Bedieneinheit 4 gegenüber der Messdose 1 vorzusehen, in welchen die Messdose 1 und die Bedieneinheit 4 eine feste Verbindung miteinander eingehen können. Der Umfang der Erfindung soll daher durch das gezeigte Beispiel nicht eingeschränkt werden.

### Bezugszeichen

- 1: Messdose
- 2: Gehäuseteil
- 3: Messwerk
- 4: Bedieneinheit
- 5: Halterung
- 6: Pumpball
- 7: Löffel
- 8: Ablassventil
- 9: Halterungsende
- 10: Halterungsende
- 11: Halterungsende
- 12: Halterungsende
- 13: Anschlussöffnung
- 14: Gummiring
- 15: Zunge
- 16: Verriegelungselement
- 17: Rasteinrichtung
- 18, 18': Löffelfläche
- 19: Platte
- 20, 20': Rasteinrichtung
- 21: Rastgegeneinrichtung
- 22: Rastgegeneinrichtung
- 23: Öffnung
- 24: Wulst
- 25: Kennzeichnung
- 26: Federbogen
- 27: Wand
- 28: Wandbereich
- 29: Ventilhals
- 30: Kordelschraube
- 31: Nut
- 32: Nase
- 33, 33': Nut
- 34, 34': Ring
- 35: Nut
- 36: Ablassschraube

## Patentansprüche

1. Handbetriebenes Blutdruckmessgerät, das eine Messdose mit einer Druckanzeige zur Messung eines Druckverlaufs und eine handbetriebene Bedieneinheit mit einer Druckerzeugungs- und einer Druckregulierungsvorrichtung (6, 7; 8) aufweist, **dadurch gekennzeichnet, dass**
die Messdose (1) und die Bedieneinheit (4) lösbar miteinander verbunden sind.

2. Handbetriebenes Blutdruckmessgerät nach Anspruch 1, wobei für die Messdose ein dieser zugeordnetes Halterungsende (9) vorgesehen ist, an das die Messdose lösbar angeschlossen ist.

3. Handbetriebenes Blutdruckmessgerät nach Anspruch 1 oder 2, **dadurch gekennzeichnet, dass** die Messdose (1) und die Bedieneinheit (4) wenigstens in zwei verschiedenen vorbestimmten Stellungen zueinander miteinander verbindbar sind.

4. Handbetriebenes Blutdruckmessgerät nach Anspruch 1 bis 3, **dadurch gekennzeichnet, dass** die Messdose (1) und die Bedieneinheit (4) in zwei zueinander um 180° verdrehten Stellungen miteinander verbindbar sind.

5. Handbetriebenes Blutdruckmessgerät nach einem der vorhergehenden Ansprüche, **dadurch gekennzeichnet, dass** eine Führungseinrichtung (15) zur Führung der Messdose (1) oder der Bedieneinheit (4) in die wenigstens zwei vorbestimmten Stellungen vorgesehen ist.

6. Handbetriebenes Blutdruckmessgerät nach einem der vorhergehenden Ansprüche, **dadurch gekennzeichnet, dass** die Messdose (1) und die Bedieneinheit (4) durch eine Schnapp-, Rast- oder Steckverbindung (17, 20, 21, 22) miteinander verbindbar sind.

7. Handbetriebenes Blutdruckmessgerät nach einem der vorhergehenden Ansprüche, **dadurch gekennzeichnet, dass** die Messdose (1) und die Bedieneinheit (4) durch ein abnehmbares Verbindungselement (16) miteinander verbunden sind.

8. Handbetriebenes Blutdruckmessgerät nach dem vorhergehenden Anspruch, **dadurch gekennzeichnet, dass** die Messdose (1) und die Bedieneinheit (4) durch das abnehmbare Verbindungselement (10) miteinander verriegelt werden.

9. Handbetriebenes Blutdruckmessgerät nach einem der vorhergehenden Ansprüche, **dadurch gekennzeichnet, dass** zwischen der Messdose (1) und der Bedieneinheit (4) ein elastisches Element (14) vorgesehen ist.

10. Handbetriebenes Blutdruckmessgerät nach dem vorhergehenden Anspruch, **dadurch gekennzeichnet, dass** das elastische Element (14) auf Pressung zwischen der Messdose (1) und der Bedieneinheit (4) angeordnet ist, wenn die Messdose (1) und die Bedieneinheit (4) in einem verbundenen Zustand sind.

11. Handbetriebenes Blutdruckmessgerät nach einem der vorhergehenden Ansprüche, **dadurch gekennzeichnet, dass** eine Kennzeichnungseinrichtung (25) zur Individualisierung vorgesehen ist.

12. Handbetriebenes Blutdruckmessgerät nach dem vorhergehenden Anspruch, **dadurch gekennzeichnet, dass** die Kennzeichnungseinrichtung (25) mit dem abnehmbaren Verbindungselement (16) anbringbar ist.

13. Handbetriebenes Blutdruckmessgerät nach einem der vorhergehenden Ansprüche, **dadurch gekennzeichnet, dass** das Verbindungselement (16) einen plattenförmigen Bereich aufweist, der wenigstens teilweise transparent ausgebildet ist.

14. Handbetriebenes Blutdruckmessgerät nach dem vorhergehenden Anspruch, **dadurch gekennzeichnet, dass** an dem Verbindungselement (16) Verbindungseinrichtungen (21) zur Verbindung mit der Messdose (1) und Verbindungseinrichtungen (22) zur Verbindung mit der Bedieneinheit (4) vorgesehen sind.

15. Handbetriebenes Blutdruckmessgerät nach einem der vorhergehenden Ansprüche, **dadurch gekennzeichnet, dass** die Messdose (1) als Austauscheinheit vorgesehen ist.

16. Handbetriebenes Blutdruckmessgerät nach einem der vorhergehenden Ansprüche, **dadurch gekennzeichnet, dass** die Druckerregulierungsvorrichtung ein Ventil (8) mit einer Ablassschraube aufweist, die durch eine Klemmverbindung am Ventil (8) gesichert ist.

17. Handbetriebenes Blutdruckmessgerät nach dem vorhergehenden Anspruch, **dadurch gekennzeichnet, dass** die Klemmverbindung durch einen Federbogen (26) erzeugt wird.

18. Handbetriebenes Blutdruckmessgerät nach einem der vorhergehenden Ansprüche, **dadurch gekennzeichnet, dass** die Druckerzeugungsvorrichtung einen Pumpball (6) aufweist, der einen nach innen gestülpten Verbindungsbereich (28) zu einer Halterung der Bedieneinheit aufweist.
